# EUROPEAN PATENT SPECIFICATION

(11) **EP 0 765 386 B1**
(45) Date of publication and mention of the grant of the patent: **10.12.2014**
(21) Application number: 95923042.6
(22) Date of filing: 13.06.1995
(51) Int. Cl.: C12N 5/07

(54) **METHODS FOR IN VIVO T CELL ACTIVATION BY ANTIGEN-PULSED DENDRITIC CELLS**
METHODE ZUR IN VIVO AKTIVIERUNG VON T-ZELLEN MIT ANTIGEN INKUBIERTEN DENDRITISCHEN ZELLEN
PROCEDES POUR L'ACTIVATION DE LYMPHOCYTES T IN VIVO PAR DES CELLULES DENDRITIQUES A IMPULSIONS ANTIGENIQUES

(30) Priority: 14.06.1994 US 259774
(43) Date of publication of application: 02.04.1997
(73) Proprietor: THE BOARD OF TRUSTEES OF THE LELAND STANFORD JUNIOR UNIVERSITY, Stanford, California 94305 (US)
(72) Inventor: ENGLEMAN, Edgar G., Atherton, CA 94027 (US); LEVY, Ronald, Stanford, CA 94305 (US); HSU, Frank, Palo Alto, CA 94303 (US); BENIKE, Claudia, Fremont, CA 94536 (US)
(74) Representative: Vossius & Partner
(86) International application number: PCT/US1995/007461
(87) International publication number: WO 1995/034638

(56) References cited:
- WO-A-91/13632
- WO-A-93/20185
- WO-A-94/02156
- SORNASSE, T. ET AL.: "Antigen-pulsed dendritic cells can efficiently induce an antibody response in vivo" JOURNAL OF EXPERIMENTAL MEDECINE, vol. 175, no. 1, 1 January 1992, page 15-21 XP002045391
- FRANK, J. ET AL.: "Antigen-pulsed dendritic cells are effective in inducing immune responses in patients with B-cell lymphoma" BLOOD, vol. 84, no. 10, Suppl.1, 15 November 1994, page 2066 XP002045392
- MACATONIA, S. E. ET AL.: "Primary proliferative and cytotoxic responses to HIV induced in vitro by human dendritic cells" IMMUNOLOGY, vol. 74, 1991, pages 399-406, XP000605686
- HSU, F.J. ET AL.: "Vaccination of patients with B-cell lymphoma using autologous antigen-pulsed dendritic cells" NATURE MEDECINE, vol. 2 , no. 1, January 1996, pages 52-58, XP000605401
- EUROPEAN JOURNAL OF IMMUNOLOGY, Volume 24, issued March 1994, FLAMAND et al., "Murine Dendritic Cells Pulsed in Vitro With Tumor Antigen Induce Tumor Resistance in Vivo", pages 605-610.
- PROC. NATL. ACAD. SCI. U.S.A., Volume 85, issued August 1988, HAUSER et al., "Activation and Expansion of Hapten- and Protein-Specific T Helper Cells From Nonsensitized Mice", pages 5625-5628.
- JOURNAL OF IMMUNOLOGY, Volume 140, Number 9, issued 01 May 1988, KAST et al., "Failure or Success in the Restoration of Virus-Specific Cytotoxic T Lymphocyte Response Defects by Dendritic Cells", pages 3186-3193.
- JOURNAL OF EXPERIMENTAL MEDICINE, Volume 172, issued August 1990, INABA et al., "Dendritic Cells Pulsed With Protein Antigens in Vitro Can Prime Antigen-Specific, MHC-Restricted T Cells in Situ", pages 631-640.
- JOURNAL OF EXPERIMENTAL MEDICINE, Volume 178, issued December 1993, PAGLIA et al., "Immortalized Dendritic Cell Line Fully Competent in Antigen Presentation Initiates Primary T Cell Responses in Vivo", pages 1893-1901.

## Description

### 1. INTRODUCTION

The present invention relates to methods of using isolated human dendritic cells to present exogenous antigens for the induction of immune responses *in vivo.* In particular, it relates to the isolation of dendritic cells from human blood, exposing the cells to lymphoma-derived immunoglobulins as antigens, and re-infusing the autologous antigen-pulsed dendritic cells into lymphoma patients to induce and/or augment a tumor-reactive immune response. The methods of the invention described herein have a wide range of applications, including but not limited to, the clinical use of antigen-pulsed dendritic cells as vaccines and/or immunotherapeutics against cancer and infectious agents such as viruses.

### 2. BACKGROUND OF THE INVENTION

### 2.1. GENERATION OF AN IMMUNE RESPONSE

The introduction of a foreign antigen into an individual elicits an immune response consisting of two major components, the cellular and humoral immune responses, mediated by two functionally distinct populations of lymphocytes known as T and B cells, respectively. The T cells may be further divided into two subsets by function and phenotype. A subset of T cells responds to antigen stimulation by producing lymphokines which "help" or activate various other cell types in the immune system. Another T cell subset is capable of developing into antigen-specific cytotoxic effector cells, being able to directly kill antigen-positive target cells. On the other hand, the B cell response is primarily carried out by secretory proteins, antibodies. Antibodies function by neutralizing antigens or in conjunction with other effector cells of the immune system in mediating antibody-dependent cellular cytotoxicity.

Helper T cells (TH) can be distinguished from classical cytotoxic T lymphocytes (CTL) and B cells by their cell surface expression of a glycoprotein marker termed CD4. Although the mechanism by which CD4⁺ TH function has not been fully elucidated, the existence of functionally distinct subsets within the CD4⁺ T cell compartment has been reported (Mosmann and Coffman, 1989, Ann. Rev. Immunol. 7:145-173). In the mouse, type 1 helper T cells (TH1) produce interleukin-2 (IL-2) and γ-interferon (γ-IFN) upon activation, while type 2 helper T cells (TH2) produce IL-4 and IL-5. Based on the profile of lymphokine production, TH1 appear to be involved in promoting the activation and proliferation of other T cell subsets including CTL, whereas TH2 specifically regulate B cell proliferation and differentiation, antibody synthesis, and antibody class switching. Some CD4⁺ T cells, like CD8⁺ CTL, appear to be capable of cytotoxic effector function as well.

A second T cell subpopulation is the classical CTL which express the CD8 surface marker. Unlike most TH, these cells display cytolytic activity upon direct contact with target cells, although they are also capable of producing certain lymphokines. *In vivo*, CTL function is particularly important in situations where an antibody response alone is inadequate. There is a preponderance of experimental evidence that CTL rather than B cells and their antibody products play a principal role in the defense against viral infections and cancer.

A salient feature of both T and B cell responses is their exquisite specificity for the immunizing antigen; however, the mechanisms for antigen recognition differ between these two cell types. B cells recognize antigens by antibodies, either acting as cell surface receptors or as secreted proteins, which bind directly to antigens on a solid surface or in solution, whereas T cells only recognize antigens that have been processed or degraded into small fragments and presented on a solid phase such as the surface of antigen-presenting cells. Additionally, antigenic fragments must be presented to T cells in association with major histocompatibility complex (MHC)-encoded class I or class II molecules. The MHC refers to a cluster of genes that encode proteins with diverse immunological functions. In man, the MHC is known as HLA. Class I gene products are found on all somatic cells, and they were originally discovered as targets of major transplantation rejection responses. Class II gene products are mostly expressed on cells of various hematopoietic lineages, and they are involved in cell-cell interactions in the immune system. Most importantly, MHC-encoded proteins have been shown to function as receptors for processed antigenic fragments on the surface of APC (Bjorkman et al., 1987, Nature 329: 506-512).

### 2.2. ANTIGEN PRESENTING CELLS

The presentation of antigens to T cells is carried out by specialized cell populations referred to as antigen presenting cells (APC). Typically, APC include macrophages/monocytes, B cells, and bone marrow-derived dendritic cells (DC). DC are sometimes also referred to as "professional" APC. APC are capable of internalizing exogenous antigens, cleaving them into smaller fragments in enzyme-rich vesicles, and coupling the fragments to MHC-encoded class I or class II products for expression on the cell surface (Goldberg and Rock, 1992, Nature 357:375-379). Since APC express both MHC-encoded class I and class II glycoproteins, they can present antigenic fragments to both CD4⁺ and CD8⁺ T cells for the initiation of an immune response.

By definition, APC not only can present antigens to T cells with antigen-specific receptors, but can provide all the signals necessary for T cell activation. Such signals are incompletely defined, but probably involve a variety of cell surface molecules as well as cytokines or growth factors. Further, the factors necessary for the activation of naive or unprimed T cells may be different from those required for the re-activation of previously primed memory T cells. The ability of APC to both present antigens and deliver signals for T cell activation is commonly referred to as an accessory cell function. Although monocytes and B cells have been shown to be competent APC, their antigen presenting capacities in *vitro* appear to be limited to the re-activation of previously sensitized T cells. Hence, they are not capable of directly activating functionally naive or unprimed T cell populations.

Although it had been known for a long time that APC could process and present antigens to T cells, it was not shown until relatively recently that small antigenic peptides could directly bind to MHC-encoded molecules (Babbit et al., 1985, Nature 317: 359; Townsend et al., 1986, Cell 44: 959). However, it is believed that normally, complex antigens are proteolytically processed into fragments inside the APC, and become physically associated with the MHC-encoded proteins intracellularly prior to trafficking to the cell surface as complexes. Two distinct pathways for antigen presentation have been proposed (Braciale et al., 1987, Immunol. Rev. 98: 95-114). It was thought that exogenous antigens were taken up by APC, processed and presented by the exogenous pathway to class II restricted CD4⁺ T cells, while the endogenous pathway processed intracellularly synthesized proteins, such as products of viral genes in virally-infected cells, for association with MHC class I proteins and presentation to CD8⁺ CTL. Although the two pathways in antigen processing and presentation may still be correct in some respects, the distinction is blurred in light of recent findings that exogenously added antigens may also be presented to class I-restricted CTL (Moore et al., 1988, Cell 54: 777).

The term "dendritic cell" refers to a diverse population of morphologically similar cell types found in a variety of lymphoid and non-lymphoid tissues (Steinman, 1991, Ann. Rev. Immunol. 9:271-296). These cells include lymphoid DC of the spleen, Langerhans cells of the epidermis, and veiled cells in the blood circulation. Although they are collectively classified as a group based on their morphology, high levels of surface MHC class II expression, and absence of certain other surface markers expressed on T cells, B cells, monocytes, and natural killer cells, it is presently not known whether they derive from a common precursor or can all function as APC in the same manner. Further, since the vast majority of published reports have utilized DC isolated from the mouse spleen, results from these studies may not necessarily correlate with the function of DC obtained from other tissue types. (Inaba et al., 1987, J. Exp. Med. 166:182-194; Hengel et al., 1987 J. Immunol., 139:4196-4202; Kast et al., 1988, J. Immunol., 140:3186-3193; Romani et al., 1989, J. Exp. Med. 169:1169-1178; Macatonia et al., 1989, J. Exp. Med. 169:1255-1264; Inaba et al., 1990, J. Exp. Med. 172:631-6640). For example, despite high levels of MHC-class II expression, mouse epidermal Langerhans cells, unlike splenic DC, are not active APC in mixed leucocyte reaction (MLR), unless cultured with granulocyte-macrophage colony stimulating factor (GM-CSF) (Witmer-Pock et al., 1987, J. Exp. Med. 166:1484-1498; Heufler et al., 1988, J. Exp. Med. 167:700-705). Most human Langerhans cells express the CD1 and CD4 markers, while freshly isolated blood DC express CD4 weakly, but not CD1. On the other hand, cultured peripheral blood DC express CD1c, but not CD4. Additionally, it has not been established the extent to which the functional characteristics observed with mouse DC are applicable to human DC, especially the DC obtained from non-splenic tissues; in part, due to inherent differences between the human and murine immune systems.

In addition, the activity of human DC in *vivo* has not been studied prior to the present invention. Although murine dendritic cells exposed to idiotype proteins of mouse lymphoma cells have been reported to induce tumor immunity *in vivo*, such success has not been demonstrated in human patients (Bohlen et al., 1991, International Publication No. WO91/13632). There is no indication in the art that such an approach is suitable for clinical use. In fact, it is well known in the art that cancer is a complicated disease, and therapy performed in animal models does not adequately predict its likelihood of success in humans. Both the efficacy and toxicity of a therapeutic regimen for human cancer can only be properly examined in a clinical setting where all variable factors are present.

### 3. SUMMARY OF THE INVENTION

The present invention relates to the use of isolated and antigen-pulsed human DC as APC in *vivo* to induce and/or augment antigen-specific immune responses. It also relates to pharmaceutical composition containing such cells.

The invention is based, in part, on the Applicants' discovery that human DC partially purified from human blood by sequential density gradient centrifugation function as potent APC in vivo in patients with B cell lymphomas. As shown in Example 6, infra, when isolated autologous DC are pulsed with immunoglobulin "custom made" from a patient's lymphoma cells and re-infused into the patient, an idiotype-specific T cell proliferative response is detected. Most importantly, the patient's tumor undergoes a substantial regression over the course of such treatment. A wide variety of uses is encompassed by the invention described herein, including but not limited to, the *in vivo* administration of antigen-pulsed DC as vaccines for priming primary immune responses and/or as immunotherapeutics for augmenting secondary immune responses. Such responses may be induced against any antigen of interest, ranging from tumor antigens such as lymphoma idiotypes, p53 tumor suppressor protein, melanoma antigen MAGE, and oncogene products to infectious agents such as human immunodeficiency virus (HIV).

### 4. BRIEF DESCRIPTION OF THE DRAWINGS

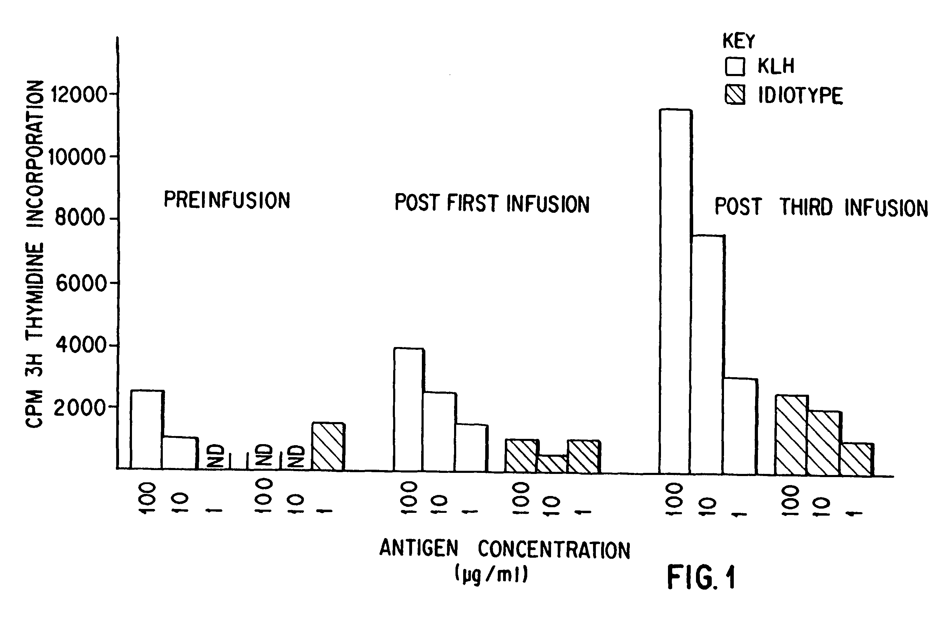

- FIG. 1: Peripheral blood lymphocytes were isolated by "FICOLL/HYPAQUE" gradient centrifugation of patient's blood drawn at the indicated point in her therapy regimen. Cells were plated at 4 x 10⁵ cells/microtiter well with KLH or autologous immunoglobulin idiotype in Iscove's modified Dulbecco's medium (IMDM) with 1% human AB serum. After 3 days of culture, the cells were split and fed with IMDM containing 5% FCS and 30 units/ml of IL-2. After a total of 5 days at 37°C in 5% CO₂ atmosphere, the cells were pulsed for 16 hours with 1 *µ*ci/well ³H-thymidine. Data are expressed as mean counts/minute of quadruplicate cultures.

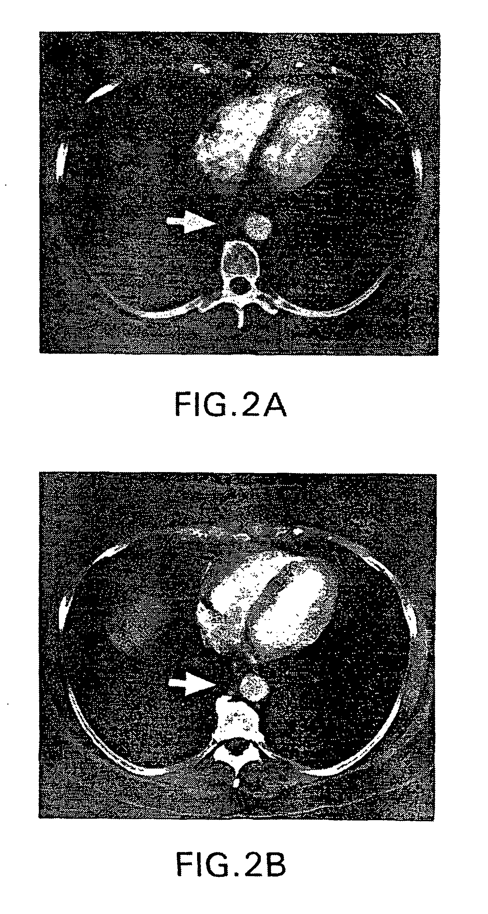

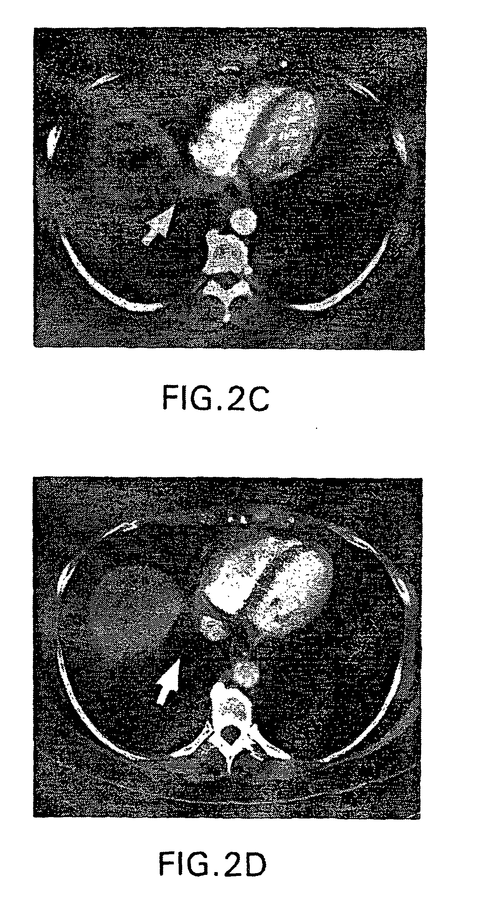

- FIG. 2A-2D: Contiguous computerized tomographic scans of the chest were obtained prior to treatment with immunoglobulin-pulsed DC and two months following the fourth immunization (optional booster). FIG. 2A and B demonstrate the regression of paraaortic adenopathy. FIG. 2C and D demonstrate the regression of a paracardiac mass and paraaortic adenopathy. Prior to treatment, these nodal masses were documented to be slowly progressing.

### 5. DETAILED DESCRIPTION OF THE INVENTION

Immunoglobulin or antibody molecules are polypeptides composed of heavy and light chains, which possess highly specific variable regions at their amino termini. The variable regions of heavy and light chains collectively form the unique antigen-recognition site of the immunoglobulin protein. These variable regions contain determinants that can themselves be recognized as antigens by the immune system, and they are referred to as idiotypes.

B-cell malignancies (B cell lymphomas, leukemias, and myelomas) are products of clonal proliferation of tumor cells synthesizing a single immunoglobulin molecule (or monoclonal antibody) with unique variable regions in the heavy and light chains. Since such tumors are monoclonal in origin, the immunoglobulin expressed by all cells of a given tumor in a patient is identical, and can be distinguished from normal B cells by virtue of its unique idiotype. B-cell lymphomas are tumors of mature lymphocytes, which generally express immunoglobulin on their cell surface. The idiotypic determinants of the surface immunoglobulin of a B-cell lymphoma can thus serve as a tumor-specific marker for the malignant clone since it is not expressed by any other tissues in the body.

Studies in animals as well as in humans have demonstrated the usefulness of the immunoglobulin idiotype as a tumor-specific antigen and as a target for passive immunotherapy in *vivo* (Campbell et al., 1990, J. Immunol. 145:1029; Campbell et al., 1988, J. Immunol. 141:3227). Active immunization against idiotypic determinants on malignant B-cells has produced resistance to tumor growth in several animal models of syngeneic tumors, as well as specific antitumor therapy against established tumors (Campbell et al., 1988, J. Immunol. 141:3227; George et al., 1988, J. Immunol. 141:2168). Moreover, preclinical studies in non-human primates have demonstrated that optimal immunization with immunoglobulin derived from human lymphomas requires conjugation of the protein to a strongly immunogenic carrier protein such as keyhole-limpet hemocyanin (KLH) and emulsification in an adjuvant.

In a recent study, several lymphoma patients with minimal disease activity were repeatedly injected with idiotype protein coupled to KLH, emulsified in an experimental adjuvant formulation (Kwak et al., 1992, New Eng. J. Med. 327:1209). Optimal immunization conditions required the use of both an immunogenic protein such as KLH and an adjuvant. In the absence of adjuvant, negligible idiotype-specific immune responses were observed. However, several patients treated with the combination of KLH-idiotype and adjuvant demonstrated an anti-idiotypic immune response in *vitro* and two patients with measurable disease experienced objective tumor regressions. However, the use of an immunologic adjuvant was associated with toxicity. Furthermore, an approved human adjuvant, alum, may not be effective in inducing anti-idiotypic responses.

The present invention demonstrates that in a patient with non-Hodgkin's, B cell lymphoma who is not eligible for bone marrow transplantation can tolerate the infusion of their own DC following *in vitro* incubation of these cells with purified immunoglobulin idiotype isolated from autologous tumor cells. The patient is shown to generate an immune response to the tumor-associated idiotype and regression of tumor burden. Since the immunogenicity of an isolated antigen such as immunoglobulin may be poor, and its induction of successful immunity requires its conjugation to an immunogenic carrier and the use of an immunogenic adjuvant, antigen-pulsed DC may replace these two requirements in presenting antigens *in vivo.* Although the specific procedures and methods described herein are exemplified using DC isolated from human blood and lymphoma-derived immunoglobulin as antigens, they are merely illustrative for the practice of the invention. Analogous procedures and techniques are equally applicable. Therefore, DC may be isolated from any source where they are found using variants of the procedure described herein, pulsed with any antigens or fragments thereof, and reinfused into patients containing cells or tissues that express the antigens for the immunologic destruction of such cells and tissues.

### 5.1. ISOLATION OF HUMAN DENDRITIC CELLS

The present invention relates to an antigen presentation system using DC for the activation of T cells in *vivo.* Due to their presence in low numbers in most tissues, DC must first be isolated and enriched. Although DC are found in both lymphoid and non-lymphoid tissues, a natural and easily accessible source of DC in man is the peripheral blood, which contains an estimate of fewer than 1 DC per 100 white blood cells.

The potency of the accessory cell function of DC in antigen presentation allows for the use of these cells in relatively small numbers when enriched, and absolute purity is not necessary for the generation of a T cell response in *vivo.* However, it is most preferable that a highly purified DC population (>90%) be used for *in vivo* administration.

DC may be isolated from a normal human or from a patient suffering from a disease. Additionally, such individuals may be treated with colony stimulating factors to increase their number of DC prior to isolation. For example, GM-CSF ("LEUKINE", Immunex Corporation, Seattle, WA) may be infused into an individual at 250 mcg/m²/day for several days up to three weeks intravenously prior to obtaining the peripheral blood mononuclear leukocytes for the purification of DC. This procedure may increase the yield of DC for antigen pulsing and subsequent infusion.

Human DC may be isolated from any tissues where they reside, using a variety of separation methods. Example 6, infra, presents variants of such methods as illustrations for isolating DC from the human peripheral blood. This procedure is principally designed to avoid the exposure of DC to antigens such as fetal calf serum, sheep red blood cells and murine monoclonal antibodies which have been used in the separation of peripheral blood leukocytes. Since DC may be able to present such proteins to T cells, even in the absence of other exogenously added antigens, conventional methods of DC isolation may lead to activation of T cells not specific for the antigens of interest, thus potentially masking the response sought. In accordance with this aspect of the invention, human peripheral blood mononuclear leukocytes (PBML) may be isolated from blood samples, particularly buffy coats or leukocytes prepared by apheresis, by "FICOLL HYPAQUE" gradient centrifugation followed by "PERCOLL" discontinuous centrifugation (Markowicz and Engleman, 1990, J. Clin. Invest. 85:955) followed by "METRIZAMIDE" (2-[3-Acetamido-5-N-methyl-acetamido-2,4,6-triiodobenzamido]-2-deoxy-D-glucose) or "NYCOPREP 1.068" NYCODENZ, N.N'-Bis(2,3 dihydroxypropl)-5-[N-(2,3-dihydroxypropyl) acetamido]-2,4,6-trilodo-isophtalamide discontinuous centrifugation. The high buoyant density (HD) fraction contains γδ and α*β*-T cells, B cells, and NK cells, whereas DC are in the low buoyant density (LD) fraction of the "METRIZAMIDE" or "NYCOPREP 1.068". The LD fraction can then be subjected to second "METRIZAMIDE" or "NYCOPREP 1.068" gradient to obtain a further enriched population of DC. DC may also be further enriched using additional protocols, depending on the level of purity required. Isolated DC can be pulsed immediately with any antigen of interest.

Alternatively, DC may be isolated by procedures involving repetitive density gradient centrifugation, positive selection, negative selection, or a combination thereof. However, the above-mentioned density gradient methods are preferred because they do not contain xenogeneic proteins in the form of mouse antibodies or sheep red blood cells which may be internalized and presented by DC prior to the addition of an exogenous antigen of interest. Positive selection methods may utilize affinity chromatography with antibodies directed to DC surface markers. Positive selection does not necessarily require the use of antibodies that recognize DC-specific determinants. For example, B cells and monocytes may be depleted first from the DC-containing fraction after density gradient centrifugation, plastic adhesion, and Fc receptor panning, then an antibody to MHC-Class II antigen can be used to positively select for DC. Negative selection includes modifications of the protocol disclosed herein, supra.

In essence, a DC-containing cell preparation may be reacted with one or more antibodies directed at cell surface antigens not expressed by DC for the removal of non-DC. Antibodies to any T cell, B cell, monocyte, and granulocyte markers may be used. Examples of such antibodies include anti-CD3, anti-CD4, anti-CD5, and anti-CD8 specific for T cells; anti-CD12, anti-CD19 and anti-CD20 specific for B cells; anti-CD14 specific for monocytes; and anti-CD16, and anti-CD56 specific for natural killer cells (Becton Dickinson, San Jose, CA and Ortho Diagnostics, NJ). These antibodies may be applied in any combination repeatedly or in a sequential manner for the enrichment of DC. Upon binding to the antibodies, the cells may be removed by adsorption to a solid surface coated with an anti-mouse antibody, as the majority of monoclonal antibodies directed at cell surface markers are of mouse origin, or if the antibodies are conjugated with biotin, the antibody-bound cells can be removed by an avidin or streptavidin-coated surface; or if the antibodies are conjugated to magnetic beads, the cells expressing antigens recognized by the antibodies can be removed in a magnetic field (Harlow and Lane, 1988, Antibodies: A Laboratory Manual, Cold Spring Harbor Laboratory Press).

### 5.2. USE OF DENDRITIC CELLS AS ANTIGEN PRESENTING CELLS

The initiation of an immune response is mediated by APC, which process complex antigens into smaller fragments by enzymatic degradation, and present them in association with MHC-encoded molecules to T cells. Although macrophages/monocytes have been studied most extensively as APC, murine DC have been shown to also possess potent accessory cell function. The present invention demonstrates that DC isolated from human blood present antigens for the activation of antigen-specific T cells *in vivo*.

### 5.2.1. ANTIGENIC SYSTEMS FOR PRESENTATION BY DENDRITIC CELLS

The potent accessory cell function of DC provides for an antigen presentation system for virtually any antigenic epitopes which T and B cells are capable of recognizing through their specific receptors. Example 6, infra, demonstrates that human DC can present immunoglobulin idiotypes as antigens to T cells *in vivo.* T cell activation is manifested by T cell proliferation in response to antigen. Hence, DC may be useful *in vivo* in presenting antigens encoded by infectious agents such as viruses, microorganisms and their products, as well as tumor antigens expressed by cancer cells (Urban and Schreiber, 1992, Ann. Rev. Immunol. 10: 617-644).

Infectious agents against which the present invention may be applicable include, but are not limited to, bacteria, parasites, fungi, and viruses. The multitudes of antigens encoded by these agents, which may be processed and presented by DC include but are not limited to, external surface proteins, and structural proteins including internal enzymes. For example, antigens encoded by any genes of the HIV genome including the env, gag, pol, nef, vif, rev, and tat genes may all be presented by DC in *vivo.* In addition, a variety of other infectious agents including hepatitis B virus, hepatitis C virus, cytomegalovirus, herpes simplex virus, varicella zoster, Staphylococcal species and Mycobacterium species are encompassed within the scope of the invention.

In addition to immunoglobulin idiotypes as tumor-specific antigens, a large number of human tumor-associated antigens have been identified by monoclonal antibodies (Reisfeld and Cheresh, 1987, Adv. Immunol. 40: 323-377). Although these cellular antigens are selectively expressed in higher quantities by certain tumor cells, it has not been established that they naturally elicit an immune response in cancer patients or can be used effectively to induce such a response. Unlike animal tumor models in which tumor-reactive T and B cells can be induced through hyperimmunization with tumor cells or tumor antigens, intact human tumor cells or oncogenic proteins may not be easily tested in humans without an approved clinical protocol. Thus, most human studies have utilized lymphocytes obtained from cancer patients whose cells presumably have been exposed to antigens expressed by their autologous tumor cells in *vivo.* However, it has been shown in some systems that tumor development is accompanied by a down-regulation of tumor specific immune responsiveness mediated by suppressor cells, and if so, T cells isolated from cancer patients may have already come under the influence of such suppression in *vivo* so as to not function in a manner similar to that of T cells obtained from tumor-immune hosts. Moreover, these attempts to activate human tumor-reactive T cells have generally used monocytes as APC, which have been shown to be much less effective than DC, especially if the T cells have not been primed adequately in *vivo* against the tumor antigens.

The antigen-pulsed DC described herein may be used as a cellular adjuvant for presenting tumor antigens *in vivo.* The potent accessory cell function of DC can present tumor antigens to T cells of cancer patients *in vivo,* whose immune response is apparently inadequate to eliminate the tumors *in vivo.* Whole tumor cells in viable or irradiated form, tumor membrane preparations, and tumor antigens purified from natural sources or expressed as recombinant products may be used to pulse DC *in vitro.*

Recently, oncogene products have been shown to be capable of inducing murine T cell activities. For example, oncogenic forms of the ras gene product p21, and the fusion product p210 of the bcr-abl gene induce T cell proliferative responses, when used to immunize mice (Peace et al., 1991, J. Immunol. 146: 2059-2065; Chen et al., 1992, Proc. Natl. Acad. Sci. USA 89: 1468-1472). Thus, oncogenic proteins which are different from their normal cellular counterparts as a result of amino acid substitutions may possess new immunogenic determinants that are recognizable by T cells. It is not necessary that such proteins be expressed naturally on the cell surface, as cytoplasmic and nuclear proteins may be processed, attached to MHC-encoded products intracellularly, and translocated to the cell surface in a complex form (Gould et al., 1989, J. Exp. Med. 170: 1051-1056). Since oncogene products are expressed in a variety of tumor types including colon cancer, leukemia and lymphoma, antigen-pulsed DC may be used to activate T cells *in vivo* against such cancers. Other molecules which are associated with various types of cancer are also encompassed by the present invention, and they include, but are not limited to, HER-2/neu gene product (United States Patent No. 4,968,603), estrogen receptor, milk fat globulin, p53 tumor suppressor protein (Levine, 1993, Annu. Rev. Biochem. 62:623), mucin (Taylor-Papadimitriou, 1990, International Publication No. WO90/05142), telomerases, nuclear matrix proteins, MART-1, MAGE-1, MAGE-2, MAGE-3 (van der Bruggen et al., 1991, Science 254:1643; Celis et al., 1994, Proc. Natl. Acad. Sci. USA 91:2105), GP100 (Bakker et al., 1994, J. Exp. Med. 179:1005), carcinoembryonic antigen, tyrosinase and papilloma viral antigens.

Bacterial, parasitic, fungal, viral, and tumor antigens of cellular or viral origin may be introduced to DC by addition to DC cultures followed by incubation, by the osmotic lysis of pinosomes after pinocytotic uptake (Moore et al., 1988, Cell 54: 777-785), or by uptake in antigen-containing liposomes. Antigens may be used as purified naturally occurring whole polypeptides, purified recombinant whole polypeptides, whole organisms or cells in viable or dead forms, protein fragments generated by enzymatic digestion, or synthetic peptides produced by solid phase chemical methods (Creighton, 1983, Protein Structures and Molecular Principles, W.H. Freeman and Co., N.Y. pp 50-60). The amount of antigens necessary for pulsing DC may vary depending on the nature, size, and purity of the molecules. In general, polypeptides may be used at 1-100 *µ*g/ml, and small peptides at 1-50 *µ*g/ml. Introduction by osmotic lysis of pinosomes requires larger amounts of proteins in the range of 200-500 *µ*g/10⁶ APC. Alternatively, exogenous genes encoding specific antigens of interest or expression vectors containing such genes or portions thereof may be incorporated into DC in expression vectors using conventional methods, including transfection, recombinant vaccinia viruses and retroviruses (Sambrook et al., 1989, Molecular Cloning: A Laboratory Manual, Cold Spring Harbor Laboratory Press). This approach causes the continual expression of integrated genes, leading to MHC occupancy by the gene products. Any of the aforementioned methods for introducing exogenous antigens into DC as well as any others commonly used by those skilled in the art are hereinafter collectively referred to as antigen pulsing of DC.

### 5.2.2. INDUCTION OF PRIMARY AND SECONDARY T CELL RESPONSES IN VIVO

The present invention relates to a method of activating an immune response in a human patient to an antigen. The method includes the isolation of human DC, preferably from the peripheral blood, pulsing the cells with an antigen in *vitro,* and then administering the antigen-pulsed cells into a patient whose cells or tissues express the antigen.

The ability of DC to process and retain antigenic fragments for several days permits their use as potent immunogens *in vivo*. DC may be pulsed with antigens according to the various methods described in Section 5.2.1, supra, washed, and administered *in vivo* as vaccines and/or immunotherapeutics for the elicitation or augmentation of a pre-existing but weak T cell response. In an effort to increase the number of isolated DC for antigen pulsing and subsequent *in vivo* administration, the isolated cells may be first expanded in number prior to incubation with antigens. It has been shown that GM-CSF and tumor necrosis factor-α induce the differentiation of human hematopoietic progenitor cells into DC (Caux et al., 1992, Nature 360:258). Thus, isolated DC may be expanded by treatment with such cytokines in culture.

Immunization with antigen-pulsed DC may increase both the magnitude and the specificity of a response. It may be desirable to repeat such immunizations at time intervals of days or weeks. The potency of DC as APC may alleviate the need of using conventional adjuvants to augment the response, although it does not preclude the use of adjuvants to further enhance immune reactivity. In addition, the antigen-pulsed DC may be administered in combination with cytokines that can maintain their number and activity for prolonged periods *in vivo*. Such cytokines include colony stimulating factors such as GM-CSF and interleukins such as IL-12.

The antigen-pulsed DC may be suspended in any known physiologic saline at a concentration sufficient to induce an immune response as detected by assays which measure T cell proliferation, T cell cytotoxicity, antibody production or reduction of the number of antigen-positive cells or tissues. A patient usually receives the total number of isolated DC pulsed with antigen. Depending on the yield of DC following each isolation procedure, a patient may be infused with several million up to several hundred million DC. The cells may be infused into autologous, HLA-matched allogeneic or even HLA-mismatched allogeneic patients.

### 6. EXAMPLE: B-CELL LYMPHOMA REDUCTION INDUCED BY AUTOLOGOUS IMMUNOGLOBULIN IDIOTYPE-PULSED HUMAN DENDRITIC CELLS

### 6.1. MATERIALS AND METHODS

### 6.1.1. CELL SEPARATION

Human DC were obtained from buffy coats of lymphoma patients after leukophoresis. PBML were isolated by "FICOLL-HYPAQUE" gradient centrifugation (Boyum, 1968, Scand. J. Clin. Lab. Invest: 21:21-29). In brief, a buffy coat was diluted with Dulbecco's PBS without divalent ions such as Ca^{2**+**} or Mg²⁺ (referred to as DPBS) up to 10 ml. 10 ml of "FICOLL" was gently underlaid into each tube and centrifuged at 1000 x g for 35 minutes at room temperature. The interface was collected and washed with DPBS three times.

To further separate various cell populations from the remaining mononuclear cell fraction, the preparation was further fractionated over a four layer discontinuous "PERCOLL" gradient (30%, 40%, 50.5% and 75%) (Pharmacia, Uppsala, Sweden) (Markowicz and Engleman, 1990, J. Clin. Invest. 85:955). Original "PERCOLL" density was prepared at 1.130g/ml DPBS and 15 ml of a 50.5% "PERCOLL" solution was made and shaken in a conical polypropylene tube to create a foam on a surface of the "PERCOLL" solution. The tube was gently underlaid with about 6.5 ml of 75% "PERCOLL". The tube was then slowly overlaid with 3 to 3.5 ml of 40% "PERCOLL" dropwise along the side of the tube which was being slowly rotated, followed by an overlay of 2.5ml of 30% "PERCOLL" in the same manner. The gradients were kept on ice for use within about 4 hours.

2.5-3x10⁸ PBML in 5 to 10 ml of DPBS supplemented with 5% human serum were overlaid onto the four layer discontinuous "PERCOLL" gradient. The cells were centrifuged at 1000 x g for 20-25 minutes at 4°C. The LD cells (monocytes) were collected from the interface over the "PERCOLL" 50.5% layer, whereas the lymphocytes and DC were collected from the interface between 75% and 50.5% layers. The collected cell fractions were diluted with DPBS at least 3 volumes and centrifuged at 1000 x g for 12 minutes at 4°C. The cells were washed twice with DPBS supplemented with 5% human serum at 400 x g for 5-6 minutes at 4°C.

The HD cells (3-7 x 10⁸/50 ml of RPMI containing 10% pooled human serum) were then cultured overnight in teflon vessels at 37°C. Thereafter, the cultured cells were subjected to gradient centrifugation in "METRIZAMIDE" (15.5%) by overlaying the cells onto 10 ml of 15.5% (wt/vol) "METRIZAMIDE" (Sigma Chemical Co.) followed by centrifugation at 650 xg for 10 min at room temperature. This fraction was further depleted of contaminating monocytes by a solid phase absorption procedure for about 20 min. on human IgG-coated petri dishes. The IgG was commercial preparation tested and approved for intravenous human use. The DC were then enriched over a second "METRIZAMIDE" gradient (14%). The HD cells from the first "METRIZAMIDE" gradient consisted of a mixture of *αβ* and γ*δ*-T cells, B cells, and NK cells. The purity of the DC obtained using this procedure were 60-90%.

Alternatively, the DC could be enriched after overnight culture by centrifugation over a "NYCOPREP 1.068" discontinuous gradient (Nycomed Pharma AS, Oslo, Norway). About 2.5 x 10⁸ cells were suspended in 15-20 ml of a solution made up of 85% DPBS, 10% human serum and 5% EDTA. This was underlaid sequentially with 4-5 ml of a solution of 50% human serum, 10% EDTA and 10% DPBS, followed by 4 ml of a solution of 75% "NYCOPREP 1.068", 24% DPBS and 1% human serum, followed by 8 ml of 100% "NYCOPREP 1.068". The cells were centrifuged at 400 xg for 13 minutes at room temperature. The interface and the pellet were collected and diluted with at least 3 volumes of DPBS containing 10% human serum, and centrifuged at 800 x g for 12 minutes at 20°C. The cells were washed twice with 10% human serum in RPMI at room temperature, and DC occupied 30-40% of the total cell population. However, these cells could be further enriched by another round of "NYCOPREP" centrifugation to obtain a LD fraction of 80-90% DC. Alternatively, the LD cells after the first "NYCOPREP" step could be negatively selected by incubation with antibody-coated petri dishes to remove CD3⁺, CD14⁺, CD16⁺ and CD20⁺ cells. The non-adherent cell population also contained 80-90% DC. However, density gradient centrifugation was preferred in order to avoid the use of xenogeneic proteins in the form of antibodies against leukocyte markers. All procedures described herein could produce a yield of 1-2.5 x 10⁶ cells from 400-500 ml of whole blood.

The purity of DC following each step of DC enrichment was assessed by staining with an anti-HLA-DR (anti-MHC class II) antibody (CA141) conjugated to fluorescein, and phycoerythrin-conjugated anti-CD14 (anti-monocyte). Cytofluorographic analysis of the entire cell population was assessed by Fluorescence Activated Cell Sorter. HLA-DR⁺ but CD14⁻ cells represented the DC population.

### 6.1.2. TUMOR IMMUNOGLOBULIN IDIOTYPE PRODUCTION

All patients had a histopathological diagnosis of non-Hodgkin's lymphoma. Immunophenotypic studies of the tumors showed that they were of B-cell origin, with the surface expression of immunoglobulin molecules composed of both heavy and light chains in a monotypic pattern. Patients were selected who had low grade B cell lymphoma and were not eligible for bone marrow transplantation. The base-line studies used to evaluate clinical disease in all patients included complete physical examination, chest radiography, routine blood counts and chemistry tests, abdominal and pelvic CT scanning, with or without bipedal lymphangiography, which were all normally part of routine clinical care of such patients. All underwent re-staging during the course of the study according to the objective procedures used at base line.

In order to cause the B cell lymphoma to secrete their immunoglobulin for purification for use as antigens, a cell suspension obtained from the tumor biopsy specimen was washed in phosphate buffered saline (PBS) and mixed with the HAT sensitive heterohybridoma B5/K6H6 (B5) in a ratio of 1:4 or 1:5 (fusion partner:tumor cells). B5 is a heterohybridoma that was produced by the fusion of the NS-1 mouse myeloma fusion partner with a human lymphoma cell. The B5 clone has lost the ability to spontaneously secrete immunoglobulin. A subclone was identified which had retained the ability to secrete immunoglobulin when fused with human B-cells. This subclone was drug-marked so that it would not survive in HAT medium. Studies by Carroll et al. (1986, J. Immunol. Methods 89:61) show B5 to be a useful fusion partner for rescuing idiotype secretion from non-secreting human B-cell tumors. No mouse immunoglobulin is produced by B5 or hybridomas derived from it.

The mixture of tumor cells and fusion partner cells was washed 2 times in Hank's balanced salt solution (HBSS). Supernatant was aspirated and the cell pellet was exposed to 40% polyethylene glycol (PEG) for 2 minutes. The PEG was diluted slowly by adding HBSS, and the cells were centrifuged. The cells were then resuspended in complete medium (RPMI Medium, 10% fetal bovine serum, glutamine 4 mM) to density of 2x10⁶ cells per ml, and 0.1 ml was then added to the wells of a microtiter plate. Twenty-four hours later after the cells had recovered from the PEG fusion, selection for rescue fusions was begun by changing the media to HAT media (complete media with the addition of hypoxanthine 10⁻⁴ M, thymidine 1.6 X 10⁻³ M and aminopterin 4 x 10⁻⁵ M). The microtiter plates containing the cells were incubated for 10-20 days at 37°C in a humidified 5% CO₂ in air incubator. The supernatants were removed from the wells and the cells were fed with the medium described above but without the aminopterin (HT medium).

An ELISA assay was used to determine which wells of the microtiter plate contained hybridomas which were secreting the rescued tumor immunoglobulin. The tumor biopsy specimen used for cell fusion contained a large predominance of malignant cells which expressed a single light chain type, either kappa or lambda. Wells secreting immunoglobulin with the heavy and light chain type corresponding to the known immunophenotype of the tumor specimen were selected and expanded.

Goat anti-human Ig heavy chain-specific reagents were diluted in 0.05 M sodium bicarbonate buffer (pH 9.5). The 96-well microtiter plates were coated with this antibody in a volume of 50 microliters per well. The plates were incubated overnight at 4°C. The plates were then washed 5 times with normal saline containing 0.05% Triton X (wash buffer). Nonspecific binding was blocked by incubating the wells with 5% non-fat milk in PBS for 1 hour and then washing 5 times with wash buffer. Hybridoma supernatants were added (50 microliters per well) and incubated for 1 hour at room temperature. The plates were again washed and developed with 50 microliters per well of horseradish peroxidase conjugated to goat anti-human Ig light chain-specific reagents. The plates were incubated for 60 minutes at room temperature. Antibody reactivity was determined by added ABTS substrate and the optic density was read at 405 nm using a microtiter plate reader.

As the hybridomas were grown, the supernatants were tested for immunoglobulin content using the ELISA described above. Dilutions of the supernatants were compared to a standard curve generated by using purified human immunoglobulin of the same isotype as that expressed on the patient's tumor. The idiotype protein was confirmed to be derived from the patient's lymphoma by cloning and comparing the DNA sequences of the variable region genes from both the lymphoma and rescue fusion. Hybridomas that constantly produced in excess of 2 micrograms per ml were expanded to large volumes (2000-5000 ml). The cell density was allowed to increase until the media was fully spent and the supernatants were collected and pooled.

The rescued tumor idiotype protein was purified from bulk supernatant using affinity chromatography. For IgM class, a murine monoclonal anti-human IgM heavy chain-specific antibody was coupled to cyanogen bromide activated sepharose B4 or an equivalent solid phase matrix at a concentration of 2-6 mg/ml of swollen beads (immunoadsorbent). Rescued tumor idiotypes of the IgA class was purified using an immunoadsorbent column consisting of a goat or rabbit anti-human IgA-specific antibody similarly coupled to a solid phase matrix. Idiotypes of the IgG class were purified using a column of protein A coupled to a solid phase. The idiotype-containing supernatant was gravity flowed through the immunoadsorbent column. After absorption, the column was washed with 200 ml of physiologic saline to remove the unbound protein. The idiotype was eluted with 0.1 M glycine-HCl buffer (pH 2.4) using absorption at 280 nm to monitor the protein effluent. The protein peak was collected and the immunoglobulin level measured using an ELISA technique or absorption at 280 nm. The protein purity was determined by SDS-PAGE.

### 6.1.3. PREPARATION OF ANTIGENS

Keyhole Limpet Hemocyanin Megathura crenulata (KLH) was obtained from Calbiochem, San Diego, California (Cat. #374805). The KLH was supplied in a form containing more than 60% protein in BES buffer and magnesium sulfate. The protein purity, which was determined by the vendor, was greater than 90%.

Prior to use, the KLH was dialyzed extensively against physiologic saline and then passed through a detoxigel column (Pierce Chemical Company, Rockford, Illinois, Cat. #20339) in PBS pH 7.4 or it was passed over a QAE Zeta Prep 15 disk (LKBm, Broma, Sweden, Cat. #224-202) using 25 mM Tris-HCl pH 8.0. A gradient of sodium chloride was used to remove the endotoxin which adhered to the column. Repeated passage of the KLH over the columns reduced the endotoxin to an acceptable level. The limulus amoebocyte lysate (LAL) assay was used to monitor endotoxin levels.

Purified idiotype specific protein and KLH were then dialyzed extensively against sterile physiologic saline. Each product was then concentrated or diluted to achieve a final concentration of 1 mg/ml in sodium chloride. The proteins were then sterile filtered through 0.45 *µ*m filters. The final concentration was determined by measuring the absorption at 280 nm or performing an ELISA on a small, expendable aliquot of the final product. The final product was aseptically filled into sterile containers under a laminar flow hood. The vials were then frozen and stored at -20 C until needed. Sterility of the final product was confirmed by a standard 5-day bacterial culture assay performed by clinical laboratory.

### 6.1.4. PULSING OF DENDRITIC CELLS WITH ANTIGENS

Enriched DC were resuspended in RPMI media at a concentration of approximately one million cells/ml. The DC were split into two independent cultures of five million cells into 24 well plates. Each set of DC was then cultured with either sterile, purified idiotype immunoglobulin protein or KLH at a concentration of fifty micrograms/ml for 4-5 hours or overnight in a humidified 37° C incubator with 5% CO2.

### 6.1.5. INJECTION OF ANTIGEN-PULSED DENDRITIC CELLS

DC pulsed with either idiotype protein or KLH were washed three times in physiologic, pyrogen-free, injection grade saline and resuspended into a volume of 100 ml in an intravenous injection administration bag. For each infusion, the patients received the total number of DC isolated from their own blood. Since each isolation yielded a different number of DC, each cell infusion contained between 5x10⁶ to 100x10⁶ antigen-pulsed DC. The patients were premedicated with acetaminophen and diphenhydramine, and received the transfusion of DC through a peripheral intravenous line or a central catheter (if available) over a period of 30-60 minutes. Vital signs were monitored continuously prior to and during the infusion. All therapy was monitored and supervised closely by a physician. Patients received similarly prepared antigen-pulsed DC for up to four times.

Any systemic reactions to the procedure were carefully noted. Systemic reactions were scored for temperature, blood pressure, and signs of bronchospasm, vasculitis, and/or immune complex formation. The risk of anaphylaxis was extremely low with the use of autologous cells.

### 6.1.6. ASSAY FOR IDIOTYPE-SPECIFIC PROLIFERATIVE RESPONSE

Proliferation assays were adapted from the techniques of Chain et al. (1987, J. Immunol. Methods 99:221). Fresh PBML, prepared by "FICOLL-HYPAQUE" gradient centrifugation, were washed and plated at a concentration of 4x10⁵ cells per well in Iscove's modified Dulbecco's medium (IMDM) with 1 percent human AB serum (IMDM-1% AB). KLH or autologous immunoglobulin idiotype at concentrations of 0 to 100 *µ*g per milliliter in IMDM-1% AB preparation was added in quadruplicate. After 3 days, the cells were divided and fed with IMDM containing 5% FCS and 30 units/ml of IL-2. After the cells were incubated for a total of five days at 37°C in an atmosphere containing 5% carbon dioxide, they were pulsed for 16 to 20 hours with ³H-labeled thymidine (1 *µ*Ci per well). Data are expressed as mean counts per minute of [³H]thymidine incorporation minus background.

### 6.1.7. ASSAY FOR ANTI-IDIOTYPIC ANTIBODIES

Microtiter plates were coated either with idiotype protein or with a panel of other immunoglobulins of the same isotype. Patient serum was serially diluted. Pretreatment samples were used as a negative control. Binding of antibodies in the patient's serum to the test panel of immunoglobulins including the patient's idiotype was detected by goat anti-human light chain-HRP antibodies specific for the light chain opposite to the patient's idiotype or by a mixture of anti-human heavy chain isotype-specific-HRP antibodies that did not react with the patient's idiotype.

### 6.2. EXAMPLES

Of three patients enrolled in the clinical study, one has completed four infusions of immunoglobulin-pulsed DC. This patient entered the study with active disease manifested by easily discernible tumor masses on the x-ray computed tomography of the chest and abdomen. After providing informed consent the patient underwent leukapheresis at the Stanford University Blood Center on three occasions at four-week intervals. DC were isolated after each leukapheresis and equal aliquots (5 x 10⁷ cells) were cultured overnight with either KLH or immunoglobulin idiotype which had been isolated from the patient's own tumor. The antigen-pulsed DC were extensively washed to remove any free immunoglobulin, suspended in isotonic saline and infused intravenously. Two weeks after each infusion, the patient received 0.5 mg of soluble idiotype protein in sterile physiologic saline subcutaneously as a boost, and a separate 0.5 mg boost of sterile KLH at a separate subcutaneous site. The cell infusions and boosts were well tolerated and two weeks following each infusion fresh lymphocytes isolated from peripheral blood were challenged in *vitro* with graded doses of either KLH or idiotype, and their proliferative activity was assayed.

Prior to DC infusion, the patient failed to respond to either KLH or immunoglobulin idiotype; however, following DC infusion the patient's lymphocytes proliferated in response to both KLH and idiotype. As shown in FIG. 1, there was little or no response to these antigens prior to the infusion of antigen-pulsed DC, there were significant proliferative responses following the initial infusion, and the responses increased following the third infusion. In addition, the patient also produced anti-idiotypic antibodies in the serum that reacted with the patient's own tumor-derived idiotype protein. Most importantly, the patient's tumor burden, which had been increasing for more than one year prior to infusion of DC was reduced by more than 50% when measured after the third infusion of antigen-pulsed DC and by nearly 100% after the fourth treatment (FIG. 2A-D).

These results demonstrate the feasibility and safety of isolating DC, pulsing them with a tumor antigen, and infusing the cells into autologous patients at repeated intervals. They further suggest that such infusions are associated with the induction of clinically significant immune responses.

## Claims

1. Use of isolated human dendritic cells which have been pulsed with an antigen in *vitro* in the manufacture of a medicament for activating an immune response to an antigen in a human patient.

2. The use of claim 1 in which the dendritic cells are isolated from human peripheral blood.

3. The use of claim 1 in which the immune response is mediated by T cells.

4. The use of claim 1 in which the immune response is mediated by B cells or their secreted antibodies.

5. The use of claim 1 in which the antigen is produced by tumor cells.

6. The use of claim 5 in which the antigen is produced by B-cell tumor cells.

7. The use of claim 6 in which the antigen is an immunoglobulin.

8. The use of claim 1 in which the antigen is a whole microorganism.

9. The use of claim 1 in which the antigen is a virus.

10. The use of claim 1 in which the antigen is a polypeptide.

11. The use of claim 1 in which the antigen is a peptide.

12. A pharmaceutical composition comprising isolated human dendritic cells which have been pulsed with an antigen *in vitro.*

13. The pharmaceutical composition of claim 12 in which the dendritic cells are isolated from human peripheral blood.

14. The pharmaceutical composition of claim 12 in which the antigen is produced by tumor cells.

15. The pharmaceutical composition of claim 14 in which the antigen is produced by B-cell tumor cells.

16. The pharmaceutical composition of claim 15 in which the antigen is an immunoglobulin.

17. The pharmaceutical composition of claim 12 in which the antigen is a microorganism.

18. The pharmaceutical composition of claim 12 in which the antigen is a virus.

19. The pharmaceutical composition of claim 12 in which the antigen is a polypeptide.

20. The pharmaceutical composition of claim 12 in which the antigen is a peptide.

## Patentansprüche

1. Verwendung isolierter, menschlicher dendritischer Zellen, die mit einem Antigen *in vitro* beladen wurden, zur Herstellung eines Medikaments zur Aktivierung einer Immunantwort auf ein Antigen in einem menschlichen Patienten.

2. Verwendung nach Anspruch 1, in der die dendritischen Zellen aus menschlichem peripherem Blut isoliert sind.

3. Verwendung nach Anspruch 1, in der die Immunantwort durch T-Zellen vermittelt ist.

4. Verwendung nach Anspruch 1, in der die Immunantwort durch B-Zellen oder durch von diesen sezernierte Antikörper vermittelt wird.

5. Verwendung nach Anspruch 1, in der das Antigen von Tumorzellen produziert wird.

6. Verwendung nach Anspruch 5, in der das Antigen von B-Zell-Tumorzellen produziert wird.

7. Verwendung nach Anspruch 6, in der das Antigen ein Immunglobulin ist.

8. Verwendung nach Anspruch 1, in der das Antigen ein ganzer Mikroorganismus ist.

9. Verwendung nach Anspruch 1, in der das Antigen ein Virus ist.

10. Verwendung nach Anspruch 1, in der das Antigen ein Polypeptid ist.

11. Verwendung nach Anspruch 1, in der das Antigen ein Peptid ist.

12. Pharmazeutische Zusammensetzung, die isolierte menschliche dendritische Zellen umfasst, die mit einem Antigen *in vitro* beladen wurden.

13. Pharmazeutische Zusammensetzung nach Anspruch 12, in der die dendritischen Zellen aus menschlichem peripherem Blut isoliert sind.

14. Pharmazeutische Zusammensetzung nach Anspruch 12, in der das Antigen von Tumorzellen produziert wird.

15. Pharmazeutische Zusammensetzung nach Anspruch 14, in der das Antigen von B-Zell-Tumorzellen produziert wird.

16. Pharmazeutische Zusammensetzung nach Anspruch 15, in der das Antigen ein Immunglobulin ist.

17. Pharmazeutische Zusammensetzung nach Anspruch 12, in der das Antigen ein Mikroorganismus ist.

18. Pharmazeutische Zusammensetzung nach Anspruch 12, in der das Antigen ein Virus ist.

19. Pharmazeutische Zusammensetzung nach Anspruch 12, in der das Antigen ein Polypeptid ist.

20. Pharmazeutische Zusammensetzung nach Anspruch 12, in der das Antigen ein Peptid ist.

## Revendications

1. Utilisation de cellules dendritiques humaines isolées qui ont été pulsées avec un antigène *in vitro* dans la fabrication d'un médicament pour activer une réponse immunitaire à un antigène chez un patient humain.

2. Utilisation selon la revendication 1 dans laquelle les cellules dendritiques sont isolées à partir de sang périphérique humain.

3. Utilisation selon la revendication 1 dans laquelle la réponse immunitaire est médiée par des lymphocytes T.

4. Utilisation selon la revendication 1 dans laquelle la réponse immunitaire est médiée par des lymphocytes B ou par les anticorps sécrétés par ceux-ci.

5. Utilisation selon la revendication 1 dans laquelle l'antigène est produit par des cellules tumorales.

6. Utilisation selon la revendication 5 dans laquelle l'antigène est produit par des cellules tumorales de lymphocyte B.

7. Utilisation selon la revendication 6 dans laquelle l'antigène est une immunoglobuline.

8. Utilisation selon la revendication 1 dans laquelle l'antigène est un microorganisme entier.

9. Utilisation selon la revendication 1 dans laquelle l'antigène est un virus.

10. Utilisation selon la revendication 1 dans laquelle l'antigène est un polypeptide.

11. Utilisation selon la revendication 1 dans laquelle l'antigène est un peptide.

12. Composition pharmaceutique comprenant des cellules dendritiques humaines isolées qui ont été pulsées avec un antigène *in vitro.*

13. Composition pharmaceutique selon la revendication 12 dans laquelle les cellules dendritiques sont isolées à partir de sang périphérique humain.

14. Composition pharmaceutique selon la revendication 12 dans laquelle l'antigène est produit par des cellules tumorales.

15. Composition pharmaceutique selon la revendication 14 dans laquelle l'antigène est produit par des cellules tumorales de lymphocyte B.

16. Composition pharmaceutique selon la revendication 15 dans laquelle l'antigène est une immunoglobuline.

17. Composition pharmaceutique selon la revendication 12 dans laquelle l'antigène est un microorganisme.

18. Composition pharmaceutique selon la revendication 12 dans laquelle l'antigène est un virus.

19. Composition pharmaceutique selon la revendication 12 dans laquelle l'antigène est un polypeptide.

20. Composition pharmaceutique selon la revendication 12 dans laquelle l'antigène est un peptide.
